# EUROPEAN PATENT SPECIFICATION

(11) **EP 3 273 853 B1**
(45) Date of publication and mention of the grant of the patent: **29.01.2020**
(21) Application number: 16714183.7
(22) Date of filing: 24.03.2016
(51) Int. Cl.: A61B 5/0488, A61B 5/00, A61B 18/14, A61B 18/24, A61B 5/20, A61N 1/36, A61B 18/00, A61B 18/02, A61B 18/18, A61N 7/00

(54) **DEVICE FOR IDENTIFYING TREATMENT SITES**
VORRICHTUNG ZUR IDENTIFIZIERUNG VON BEHANDLUNGSSTELLEN
DISPOSITIF D'IDENTIFICATION DE SITES DE TRAITEMENT

(30) Priority: 25.03.2015 US 201562137979 P
(43) Date of publication of application: 31.01.2018
(73) Proprietor: Boston Scientific Scimed, Inc., Maple Grove, MN 55311 (US)
(72) Inventor: NAGALE, Sandra, Bolton, MA 01740 (US); CLARK, Bryan, Forest Lake, MN 55025 (US); HE, Ding, Sheng, Tyngsboro, MA 01879 (US); SWANSON, Lynne, Edina, MN 55424 (US); WERNER, Dennis, Big Lake, MN 55309 (US); HARRAH, Timothy, Cambridge, MA 02139 (US)
(74) Representative: Pfenning, Meinig & Partner mbB
(86) International application number: PCT/US2016/023881
(87) International publication number: WO 2016/154371

(56) References cited:
- WO-A1-2013/188640
- WO-A2-2013/160772
- US-A- 5 494 042
- US-A1- 2004 152 997
- US-A1- 2013 053 916
- US-A1- 2013 090 640
- US-A1- 2015 011 843

## Description

### Field of the Disclosure

The disclosure relates generally to utilizing electrodes as diagnostic tools, and, more specifically, to devices for identifying treatment sites.

### Background

Portions of the human body sometimes fail to function properly. Often the cause of the malfunction is limited to a specific area or location, and not the entire malfunctioning portion (e.g., an entire organ, an entire body tract, etc.). It can be unnecessary, wasteful, or even dangerous to treat the entire organ, tract, etc., because healthy and/or properly functioning areas will be treated too. For example, a patient's digestive tract may not be functioning properly, but the cause may only be a small portion of the small intestine. Treating the entire digestive tract, including properly functioning portions, may cause the properly functioning portions (e.g., the entire digestive tract except the small portion of the small intestine) to function improperly. In another example, only certain portions of the bladder may cause an overactive bladder condition, and thus only those portions may require treatment.

Overactive Bladder or OAB is one of the factors that can result in urinary incontinence conditions. OAB is a chronic urological condition characterized broadly as the involuntary and uncontrollable urge felt by a subject to relieve the bladder, leading to abnormally high urinating frequency. Such conditions may occur due to frequent and spontaneous contractions of the detrusor muscle of the pelvic region of a subject.

Overactive bladders often exhibit localized changes in detrusor morphology, likely originating from defects on cellular and multicellular level. Such cell related deviations may be attributed to local pathological changes in the muscle condition or topology that may contribute to anomalies in the functionality of the detrusor muscle on a macroscopic scale. These changes are correlated to the observed local pathological changes in the muscle (e.g. patchy denervation, fibrosis, increased amount of connective tissue between muscle bundles) which may contribute to abnormal function of the detrusor muscle on a macroscopic scale. Moreover, some studies suggest that abnormal activity may originate from one or more distinct anatomical areas of the bladder such as the dome, internal sphincter, or the trigone.

Current solutions for overactive bladder treatment (e.g. systemic drugs, nerve stimulation, and Botox injections) target the abnormal function of the entire bladder and may not specifically address local and anatomical abnormalities, thereby indicating a need for methods and devices capable of identifying and providing therapy to specific areas where local bladder abnormality originates. In addition, current treatments, like Botox injections, need to be repeated as the effect wears off over time. Further, overtreatment with Botox leads to urinary retention which requires self-catheterization in order to void. As such, existing solutions for OAB may fail to properly address local and anatomical abnormalities of the detrusor muscle, thereby indicating the need for alternative therapies for local bladder abnormalities.

Similarly, other malfunctions within the bladder, other organs, the urinary tract, or other tracts may be due to local and anatomical abnormalities, but current solutions fail to identify the location of these abnormalities or treat only these specific locations.

For example, document WO 2013/188640 A1 discloses a system for modulating bladder function and a system for evaluating the electrophysiological function of a bladder is disclosed. Moreover, WO 2013/188640 A1 discloses methods for performing a surgical procedure on a bladder, a system for performing surgical procedures in a minimally invasive manner, and an implantable device for monitoring and/or performing a neuromodulation procedure on a bladder.

Document WO 2013/160772 A2 describes regions of tissue having reduced electrical propagation which are created in a bladder to affect its electrical or mechanical properties. To create these tissue regions, a tubular device is advanced through the urethra leading to the interior of the bladder, a distal expandable structure of the device is expanded to contact the inner wall of the bladder, and electrodes or other active energy delivery elements of the device are activated to deliver ablation energy. The electrodes or other active energy delivery elements are disposed over the expandable structure which is shaped to conform to the interior of the bladder. The inner wall of the organ is ablated in a predetermined pattern. The same or other electrodes disposed over the expandable structure can be used to electrically map the bladder. This map of electrical activity can be used to create the predetermined pattern.

Document US 2013/090640 A1 discloses a method for treating a urinary system. The method includes positioning one or more treatment units with respect to an organ wall. The one or more treatment units may be configured to deliver thermal energy. The method further includes delivering energy through the one or more treatment units to heat tissue of the organ wall.

Document US 2015/011843 A1 describes a catheter system for sympathectomy procedures and a catheter system for micro ablation procedures. Moreover, methods for performing a surgical procedure and a system for performing surgical procedures in a minimally invasive manner.

Document US 2013/053916 A1 discloses a medical device and associated method which classify candidate pacing electrode sites for delivering pacing pulses to a patient's heart. A first morphology template is established and stored in a memory of the device. A processor is configured to determine a cardiac signal morphology in response to delivering pacing pulses at a candidate pacing site in a first heart chamber. The processor compares the determined cardiac signal morphology to the first morphology template. The pacing site in the first heart chamber is classified in response to the comparing of the determined cardiac signal morphology and the first morphology template.

Document US 5 494 042 A discloses systems and methods which examine heart tissue morphology using three or more spaced apart electrodes, at least two of which are located within the heart in contact with endocardial tissue. The systems and methods transmit electrical current through a region of heart tissue lying between selected pairs of the electrodes, at least one of the electrodes in each pair being located within the heart. The systems and methods derive the electrical characteristic of tissue lying between the electrode pairs based, at least in part, upon sensing tissue impedances. The systems and methods arrange the derived tissue electrical characteristics into groups of equal electrical characteristics. The systems and methods display the groups of equal electrical characteristics in spatial relation to the location of the examined tissue regions. The systems and methods are intended to make possible the use of multiple endocardial electrodes for taking multiple measurements of the electrical characteristics of heart tissue. Multiplexing can be used to facilitate data processing. The systems and methods are further intended to make possible the identification of regions of low relative electrical characteristics, indicative of infarcted tissue, without invasive surgical techniques.

The disclosure of US 2004/152997 A1 relates to a method and a system for determining a condition of a selected region of tissue to facilitate the location of surgical resection margins. Electropotential and impedance are measured at one or more locations in an area of the body where tissue is to be removed. An agent may be introduced into the region of tissue to enhance electrophysiological characteristics of that tissue. The condition of the tissue is measured by electropotential and impedance profile. Differences in the profile are used to determine the borders between normal and abnormal tissue so as to facilitate what tissue to resect. Methods and apparatus are also disclosed to determine the efficacy of various therapies.

The devices and methods of the current disclosure may rectify some of the deficiencies described above or other deficiencies in the art.

### SUMMARY

The invention is defined in the claims. Aspects, embodiments or examples of the present disclosure not falling under the scope of the claims do not form part of the invention. Aspects of the present disclosure provide methods and devices for identifying treatment sites. It is to be understood that both the foregoing general description and the following detailed description are exemplary and explanatory only and are not restrictive.

In one example, a device may include a plurality of electrodes, a memory device configured to store an instruction for evaluating electrical activity; and a processor configured to: access the instruction from the memory; direct, with the instruction, an electrical energy source configured to generate a pacing stimulus through a first pair of the plurality of electrodes; measure, with the instruction, a resulting electrical activity at one or more of the plurality of electrodes; and identify, with the instruction, at least one treatment site based on the resulting electrical activity.

Examples of the device may additionally and/or alternatively include one or more other features. For example, the pacing stimulus may be a low frequency pacing stimulus. The pacing stimulus may be a high frequency pacing stimulus. The processor may be further configured to direct, with the instruction, the electrical energy source to generate the pacing stimulus through a first portion of the plurality of electrodes; measure, with the instruction, a first resulting electrical activity at the first portion of the plurality of electrodes; and identify, with the instruction, a first pair of the plurality of electrodes based on the first resulting electrical activity. The processor may be further configured to initiate, with the instruction, a therapy to the at least one treatment site. The therapy may include application of at least one of a radio frequency energy, an ultrasound energy, a laser energy, a cryoablation, a microwave ablation, a Botox injection, a neurolytic agent, an optical energy, an irreversible electroporation, and a hydrogel injection. The processor may be further configured to communicate, with the instruction, the resulting electrical activity to a display.

In another example, a device may include a plurality of electrodes, a memory device configured to store an instruction for evaluating electrical activity, such as electrical signals, and a processor configured to execute the instruction to perform a method. The method may include measuring a spontaneous electrical activity at the plurality of electrodes, and identifying a pair of the plurality of electrodes with the highest spontaneous electrical activity.

Examples of the device may additionally and/or alternatively include one or more other features. For example, the method may further include instructing an electrical energy source to activate the pair of the plurality of electrodes based on an amount of spontaneous electrical activity, such as the highest amount. The method may further include: instructing an electrical energy source to generate a pacing stimulus through the pair of the plurality of electrodes with, for example, the highest amount of spontaneous electrical activity; measuring a resulting electrical activity with one or more of the plurality of electrodes; identifying at least one treatment site based on the resulting electrical activity; and initiating a therapy to the at least one treatment site, such as the site with a highest amount of the resulting electrical activity. The pacing stimulus may be a low frequency pacing stimulus. The pacing stimulus may be a high frequency pacing stimulus. The therapy may include application of at least one of a radio frequency energy, an ultrasound energy, a laser energy, a cryoablation, a microwave ablation, a Botox injection, a neurolytic agent, an optical energy, an irreversible electroporation, and a hydrogel injection. The method may further include communicating the resulting electrical activity to a display.

In another example, a method may include engaging a plurality electrodes with a plurality of locations on or adjacent an interior wall of a patient; generating a pacing stimulus through a first pair of the plurality of electrodes and measuring a resulting electrical activity; and identifying at least one treatment site based on the resulting electrical activity.

Examples of the method may additionally and/or alternatively include one or more other features. For example, the interior wall may a bladder wall. Generating the pacing stimulus may include generating a low frequency pacing stimulus. Generating the pacing stimulus may include generating a high frequency pacing stimulus. The method may further comprise generating the pacing stimulus through a first portion of the plurality of electrodes and measuring a first resulting electrical activity; and identifying the first pair of the plurality of electrodes based the first resulting electrical activity. The method may further include initiating a therapy to the at least one treatment site. Initiating the therapy may include applying at least one of a radio frequency energy, an ultrasound energy, a laser energy, a cryoablation, a microwave ablation, a Botox injection, a neurolytic agent, an optical energy, an irreversible electroporation, and a hydrogel injection. In addition, the method may include displaying the resulting electrical activity.

In another example, a method may include engaging a plurality electrodes with a plurality of locations on or adjacent a bladder wall, generating a pacing stimulus through a first portion of the plurality of electrodes and measuring a resulting electrical activity with a second portion of the plurality of electrodes, and identifying at least one treatment site based on the resulting electrical activity.

Examples of the method may additionally and/or alternatively include one or more other features. For example, the method may include identifying a first pair of electrodes based on the resulting electrical activity. The method may additionally include initiating a therapy to the at least one treatment site. Initiating the therapy may include applying at least one of a radio frequency energy, an ultrasound energy, a laser energy, a cryoablation, a microwave ablation, a Botox injection, a neurolytic agent, an optical energy, an irreversible electroporation, and a hydrogel injection. The method may additionally include adjusting the therapy until the resulting electrical activity reaches a predetermined amount, for example, by titrating the therapy higher until the resulting electrical activity reaches a threshold.

### BRIEF DESCRIPTION OF THE DRAWINGS

The accompanying drawings, which are incorporated in and constitute a part of this specification, illustrate exemplary aspects of the present disclosure and together with the description, serve to explain principles of the disclosure.
FIG. 1 illustrates a system for identifying treatment sites and treating a medical condition in accordance with principles of the present disclosure;
FIG. 2 is a block diagram of an exemplary method of identifying treatment sites and treating an interior wall of a patient in accordance with principles of the present disclosure;
FIG. 3 is a schematic view of an exemplary treatment device within the patient in accordance with principles of the present disclosure;
FIGS. 4-6 are graphical representations of electrical activity illustrating exemplary outputs to an interface in accordance with principles of the present disclosure; and
FIG. 7 is an exemplary alternative leg of an electrode array of the system for identifying treatment sites and treating a medical condition in accordance with principles of the present disclosure.

### DESCRIPTION OF EXEMPLARY EMBODIMENTS

The present disclosure relates generally to methods and devices for identifying treatment sites. Specifically, the disclosure relates to measuring electrical activity at a plurality of locations on an interior wall of a patient, and identifying at least one treatment site from the measurements. The method disclosed herein may then apply one or more pacing stimuli to a single electrode (or electrode pair) at one location while measuring/sensing a resulting electrical activity at another location, for example, with the remaining electrodes. The measured electrical activity and/or the results of applying a pacing stimulus to one electrode may identify the at least one treatment site. In some implementations, a therapy may be applied to the treatment site(s) after identification.

Reference is now made in detail to examples of the present disclosure, examples of which are illustrated in the accompanying drawings. Wherever possible, the same reference numbers will be used throughout the drawings to refer to the same or like parts. The term "distal" refers to a position farther away from a user end of the device. The term "proximal" refers a position closer to the user end of the device. As used herein, the term "approximately" indicates a range of values within +/- 5%.

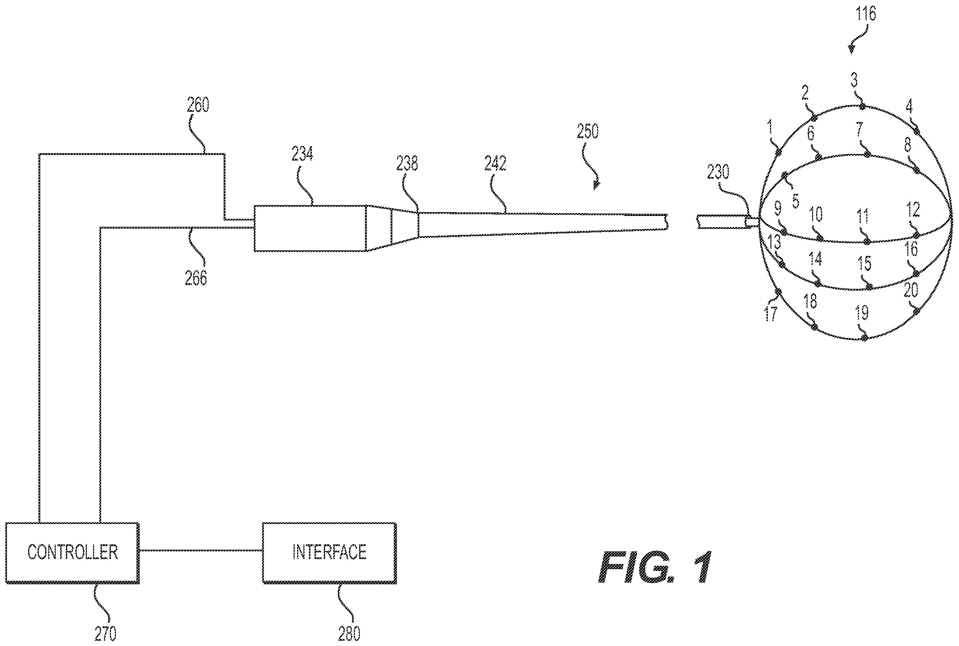

FIG. 1 illustrates an exemplary medical device 250. Medical device 250 may include a catheter 242, a handle portion 234, and an electrode array 116. Catheter 242 may have a proximal end 238 and a distal end 230. Handle portion 234 may be disposed at proximal end 238 of catheter 242. Electrode array 116 may be disposed within catheter 242 or, as shown in FIG. 1, may be disposed at distal end 230 of catheter 242. As shown in the example illustrated in FIG. 1, electrode array 116 may include one or more electrodes 1-20 uniformly distributed over electrode array 116 to supply electricity, detect electrical signals, and/or deliver therapeutic treatment to an organ of the patient. Electrode array 116 many include any number of electrodes, in any configuration. For example, array 116 is shown as being spherical in FIG. 1, although any geometric shape is possible, including those confirmative to an interior cavity of a body. Electrodes 1-20 may be capable of measuring electric current or other parameters such as impedance and/or temperature. The same electrodes may be capable of delivering pacing energy. In some examples, medical device 250 may be capable of applying therapy, such as radio-frequency ("RF") energy, ultrasound energy (e.g., high intensity focused ultrasound), laser energy, cryoablation, microwave ablation, Botox injections, neurolytic agents, optical energy sources, irreversible electroporation, hydrogel injections, and/or other suitable technologies that affect the reactivity of nerve(s). Electrode array 116 may be made of, for example, stainless steel, metal-polymer composites, and/or metal alloys of nickel, titanium, copper cobalt, vanadium, chromium, and iron. In one example, the material forming electrode array 116 may be a superelastic material such as nitinol, which is a nickel-titanium alloy.

Catheter 242 may be a tube made from any suitable biocompatible material known to one of ordinary skilled in the art having sufficient flexibility to traverse a urinary tract. Such materials may include, but are not limited to, rubber, silicone, silicone rubber, synthetic plastics, and/or polymers, such as a polyolefin triblock polymer like poly(Styrene-block-IsoButylene-block-styrene)(SIBS), latex, polyurethane, polytetrafluoroethylene (PTFE), ethylene tetrafluoroethylene (ETFE), perfluoroalkoxy (PFA), polyether ether ketone (PEEK), high density polyethylene (HDPE), and/or polypropylene (PP). In another example, the material forming catheter 242 may be a superelastic material such as nitinol, which is a nickel-titanium alloy. In yet another example, catheter 242 may include one or more metals and/or allows.

Catheter 242 may have any cross-sectional shape and/or configuration and may be any desired dimension that can be received in the lower urinary tract. An outer sheath (not shown) may surround catheter 242. The outer sheath may be constructed from an insulating polymer material such as polyamide, polyurethane, or any other suitable material. At least a portion of the outer sheath, such as a distal portion, may be deflectable and/or steerable. Catheter 242 may also include one or more lumens extending from proximal end 238 of the catheter 242 to distal end 230 of the catheter 242. The lumens may have any size, cross-sectional area, shape, and/or configuration.

In one example, medical device 250 may attach to or may include a computer system including a controller 270 and/or an interface 280. Controller 270 may include signal processing and/or an electrical energy source in or connected to handle 234 of medical device 250 via wires 260 and wires 266, respectively. In some implementations, medical device 250 may include other components, including, but not limited to, an acoustic transducer, a fluid source, a coolant source, and/or a laser source.

Controller 270 may control and/or allow an operator to control the operation of various components of medical device 250. In some implementations, controller 270 may include, for example and without limitation, a processor and a memory. The memory may include any type of random access memory (RAM) or read-only memory (ROM) embodied in a physical storage medium, such as magnetic storage including floppy disk, hard disk, or magnetic tape; semiconductor storage such as solid state disk (SSD) or flash memory; optical disc storage; cloud storage; Digital Imaging and Communications in Medicine (DICOM) compatible storage; or magneto-optical disc storage. Software may include one or more applications and an operating system. According to one aspect, the memory may store processor-readable instructions, such as instruction for evaluating electrical activity. The processor may execute those instructions to perform one or more method steps. The processor may, for example, instruct the electrical energy source to activate, measure electrical activity from electrodes 1-20, and/or identify a treatment site based on the electrical activity.

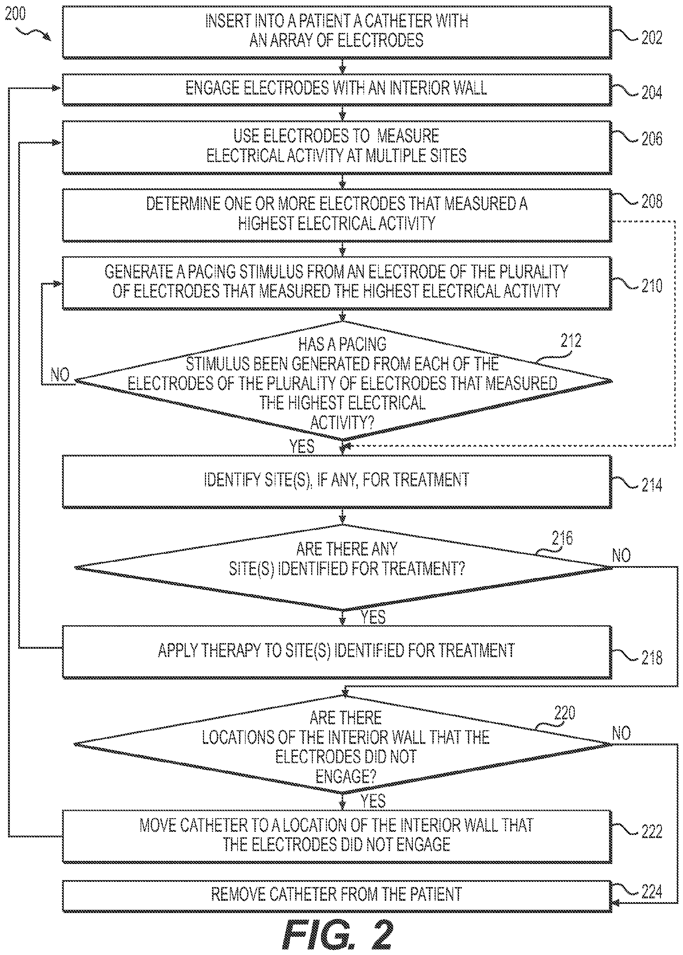

In some implementations, controller 270 may control the steering of catheter 242. In one example, controller 270 (or the processor within controller 270) may control the frequency, pattern, and destination of electrical energy from the electrical energy source to one or more of electrodes 1-20. Controller 270 (or the processor within controller 270) may receive and/or process electrical signals received from medical device 250, including from electrode array 116 and/or any of electrodes 1-20. Controller 270 (or the processor within controller 270) may also perform a variety of tasks depending on the nature of medical device 250 such as determining the geometrical characteristics of a region of interest, generating images of the region of interest and/or graphical representations of received electrical signals for output to a display (not shown) of the interface 280, or controlling the delivery of therapy to the treatment site(s). Controller 270 (or the processor within controller 270) may communicate with interface 280. Such communication may include information related to received signals and/or processed signals. Controller 270 (or the processor within controller 270) may perform, in whole or in part, exemplary methods described in further detail with respect to method 200 of FIG. 2. In some implementations, controller 270 (or the processor within controller 270) may be connected to interface 280. The interface 280 may communicate to controller 270 (or the processor within controller 270) input commands from an operator, including commands used to control and/or provide data to an energy supply source, electrodes, and/or any other components of medical device 250. Interface 280 may include user input device(s), including but not limited to any type or combination of input/output devices, such as a display monitor, touchpad, touchscreen, microphone, camera, keyboard, and/or mouse. Interface 280 may include a display screen for output to an operator. The display screen may display, for example, graphical representations of electrical signals received from one or more of electrodes 1-20, communicated to and processed by controller 270 (or the processor within controller 270).

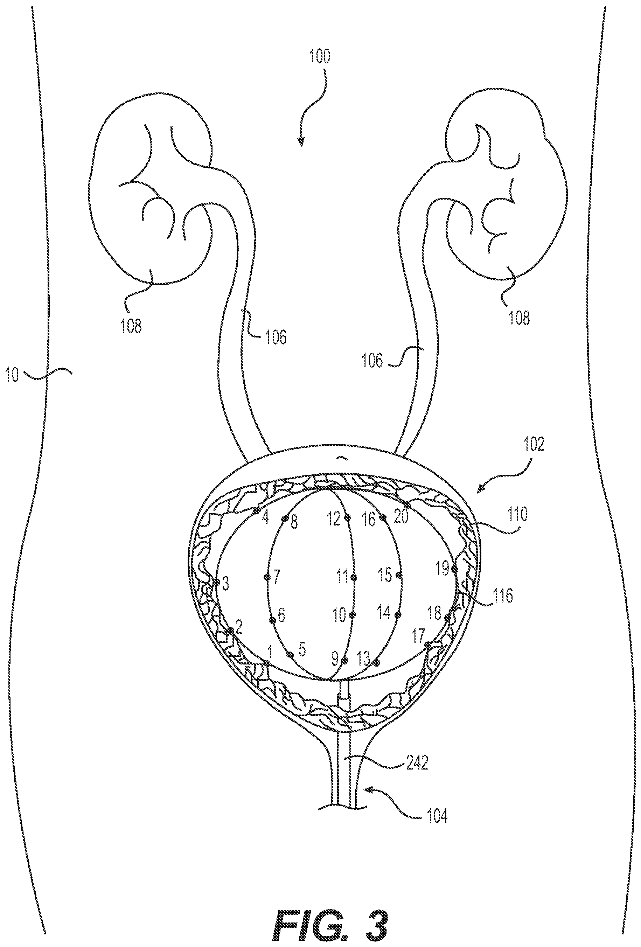

FIG. 2 is a process flow diagram of an exemplary method 200 for identifying and treating treatment site(s) within a patient. For purposes of discussion, method 200 will be described using medical device 250 of FIGS. 1 and 3, and urinary tract 100 of FIG. 3, but method 200 is not intended to be limited thereto. For example, in some implementations, the analyzed and/or treated interior wall of the patient is in another area of the body (e.g., the digestive tract). As shown in FIG. 2, method 200 includes steps 202, 204, 206, 208, 210, 212, 214, 216, 218, 220, 222, and 224. However, it should be noted that method 200 may include more or fewer steps as desired for a particular implementation and the steps may be performed in any order. In an example, one or more of the above-listed steps of method 200 may be executed by an operator, medical device 250, the processor within controller 270, controller 270, interface 280 and/or processor within interface 280 of FIG. 1, as described above. It is understood that references to the controller 270 and/or interface 280 may include reference to one or more processors therein. However, method 200 is not intended to be limited thereto, and the steps of method 200 may be performed by any party, module, device, and/or server.

Method 200 begins in step 202, which may include inserting into a patient a catheter with an array of electrodes. For example, FIG. 3 illustrates an exemplary schematic view of step 202, inserting catheter 242 with electrode array 116 into patient 10. FIG. 3 is a schematic view of an exemplary embodiment of the present disclosure, implemented for and in a urinary tract 100 of a patient 10. While this disclosure relates to the use of the disclosed system in the urinary tract of a human subject, it is understood that the features of this disclosure could be used in other locations (other organs and tissue) within a patient. The urinary tract 100 includes, among other structures, a bladder 102 that is in fluid communication with a urethra 104. Bodily fluid, such as urine, travels down from kidneys 108 to the bladder 102 via ureters 106. Muscles (not shown) in the walls of the ureters 106 tighten and relax to force the bodily fluid downward and away from the kidneys 108. The bladder 102 generally accumulates the bodily fluid, which is then discharged from the body through urethra 104. The bladder 102 includes openings for the left ureter and the right ureter, respectively, for receiving bodily fluid from the kidneys 108. In some implementations, step 202 of method 200 may be preceded by filling bladder 102 to a volume of between approximately 20% and approximately 99% of its full capacity (i.e., threshold volume). A threshold volume of bladder 102 may be determined in previously performed urodynamic studies of the patient.

In some implementations, electrode array 116 may be inserted into the body through the urethra 104 to bladder 102 in a contracted configuration (not shown). For example, electrode array 116 may be located within a lumen of catheter 242 during insertion and then exit the distal end 230 of catheter 242 and open to an expanded configuration (FIG. 3) once the distal end 230 is in the desired position. Electrode array 116 may be disposed on the distal end of an elongate member. The elongate member may be moveably disposed within a lumen of the catheter.

Electrode array 116, as shown in the drawings, is merely exemplary. Electrodes may be delivered to a region for diagnosis in any way. Other exemplary configurations are described in U.S. Patent Application No. 13/535,741, filed on June 28, 2012 and U.S. Patent Application 14/211,440, filed on March 14, 2014. Electrode array 116 may include any number of legs (e.g., the leg on which electrodes 1-4 are disposed or the leg on which electrodes 17-20 are disposed), including but not limited to 1-10 legs. Some or all of the legs may have free distal ends (e.g., the distal end of electrode array 116 may be open).In some examples, the electrode array may alternately be a single lead. The electrode array may be straight when loaded into the catheter. As the electrode array exits the catheter into the bladder, the electrode array may transform (e.g., due to the use of shape-memory material) into a spiral helix that expands to fit the bladder.

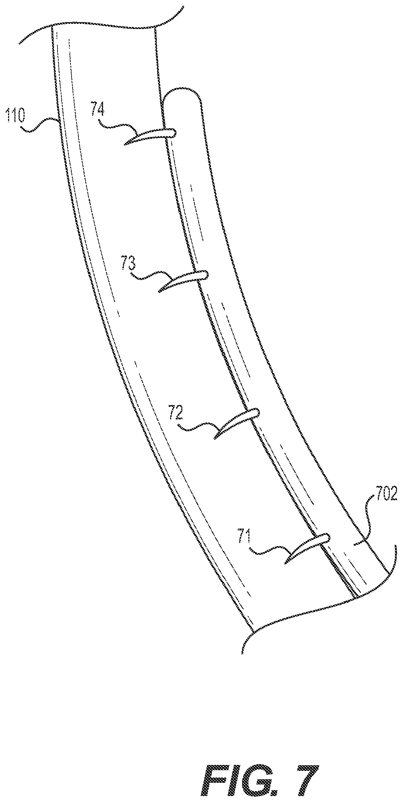

Electrodes may be located on or in the legs. In some examples, the electrodes are configured as needles. Needle electrodes may be disposed within a cavity in a leg. The cavity may be defined by a suction passage extending through the leg, and an opening between the outer surface of the leg and the passage. The opening may be on a side of the leg. The needle electrode may be selectivity extended and retracted across the opening, in directions parallel to a longitudinal axis of the leg. Tissue of the interior wall (e.g., bladder wall 110) may be suctioned into the opening to temporarily hold the interior wall against the leg. The needle electrode may be extended across the opening to a position on or adjacent the tissue. For example, the needle electrode may pierce the tissue. Alternatively, as illustrated in FIG. 7, needle electrodes 71, 72, 73, and 74 may extend from a leg 702 (e.g., radially outward of the leg 702) and into the interior wall (e.g., bladder wall 110). The needle electrodes 71, 72, 73, and 74 may be flexible. The needle electrodes 71, 72, 73, and 74 may be straight. Alternatively, the needle electrodes 71, 72, 73, and 74 may be hook-shaped to enhance their ability to hold the interior wall. The needle electrodes 71, 72, 73, and 74 may be capable of delivering Botox or other pharmaceutical agent(s) to the bladder wall 110. Additionally or alternatively, the needle electrodes may be capable of delivering a cooling substance such as saline, to prevent the tissue immediately adjacent to the each needle electrode from over-heating and/or charring when energy is applied. Each of the needle electrodes 71, 72, 73, and 74 may be connected to a tubing that would lead to a source of pharmaceutical agent(s), cooling substances, and/or any other desired, infusible material. The source may be a separate device. Each of the needle electrodes 71, 72, 73, and 74 may have individual wiring connecting the electrodes to a device capable of delivering and/or measuring electrical energy, including, for example, controller 270 of FIG. 1. In some examples, needle electrodes 71, 72, 73, and 74 may be retractable. Needle electrodes 71, 72, 73, and 74 initially disposed within leg 702 and then extended outward of leg 702 as shown in FIG. 7. Needle electrodes 71, 72, 73, and 74 may be "pushed" out of leg 702 in any suitable way, including, but not limited to, push/pull wires, slide blocks, and inflatable balloons. For example, needle electrodes 71, 72, 73, and 74 may be pushed out of leg 702 by inflating a balloon (not shown) disposed within leg 702.

Returning now to method 200, once catheter 242 with electrode array 116 is introduced into the bladder 102 in step 202, method 200 may proceed to step 204. Step 204 may include engaging each electrode with a location on or adjacent an interior wall. For example, once in the desired position, electrode array 116 may transition to an expanded configuration wherein the electrodes engage with an interior wall (e.g., bladder wall 110 of bladder 102). In some implementations, a balloon (not shown) may be inflated within electrode array 116 to expand it. Alternatively, aspects of the electrode array may include memory-shape material, such as nitinol, to transition electrode array 116 to the expanded configuration.

For example, as shown in FIG. 3, electrode array 116 may be expanded so that electrodes 1-20 are on or adjacent bladder wall 110. Electrodes 1-20 may engage bladder wall 110. For example, each electrode pair (e.g., 1-2, 3-4, etc.) may conductively engage bladder wall 10 so as to apply electrical current and/or measure electrical activity at a treatment site on the interior wall of the patient.

In step 206, one or more of the plurality electrodes may be used to measure spontaneous muscle activity at a plurality of locations on or adjacent the interior wall (e.g., any location in which the electrodes engage the interior wall). In the example illustrated in FIGS. 1 and 3, electrode array 116 may include electrodes 1-20. In some examples, measurements may be performed between adjacent electrode pairs (e.g., 1-2, 3-4, etc.). In other examples, measurements may be performed between non-adjacent electrode pairs. The measurements may be performed simultaneously at all electrode pairs or the measurements can be conducted with different portions of the plurality of electrodes at different times. To ensure measurements of electrical activity are not a product of a motion, physiological (e.g., myogenic) or extraphysiological (e.g., instrumentation, external noise, adequate grounding, etc.) artifact, each electrode measurement may be performed at normal and opposite polarity. In some aspects, the measurements may be repeated one or more additional times after array 116 has been repositioned to even further ensure that the area is active and the signals not artifact. Additionally or alternatively, one or more filters may be used to enhance the accuracy of the measurements by filtering out the artifact(s).

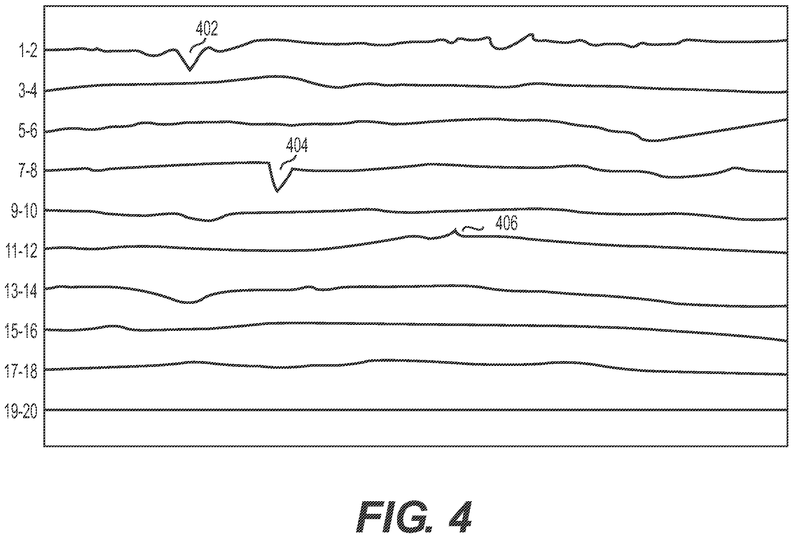

Electrodes may measure electrical activity and communicate resulting electrical signals to controller 270 (or the processor within controller 270) of FIG.1. In some examples, controller 270 (or the processor within controller 270) may process these electrical signals and/or output the signals for display at interface 280. FIG. 4 illustrates exemplary spontaneous electrical activity measured in step 206 of FIG. 2 and/or displayed at interface 280 of FIG. 1.

In some implementations, a "heat map" may be created based on the muscle activity and/or nerve activity measured in step 206. For example, electrode(s) with increased electrical signaling may be noted by measuring the sum of signal intensity at each electrode over time to create an intensity map (i.e., heat map). The locations of the interior wall that engage with these electrodes may be referred to as "hot spots," (e.g., where the electrical signal is the most intense over time). In some examples, to create a heat map, signal measurements may last seconds, minutes, etc. Heat maps, including identified hot spots, may be displayed on, for example, interface 280 of FIG. 1. If the device is configured with a GPS-like sensor (e.g., impedance or electro-magnetic technology used for mapping devices/software), the data from the device 250 may be used by mapping software to develop a 3D map of the regions where responses have been acquired and regions that have been treated. For example, the 3D map may be capable of showing the electrical signal/wave conductions, a consequential conduction direction alternation or elimination post energy, and/or an application of a pharmaceutical agent.

In step 208, one or more of the plurality of electrodes that measure an amount of electrical activity, such as the highest relative electrical activity, may be determined. In some examples, these electrodes may be determined based on the electrode signals received and heat maps and/or hot spots described above. This determination may be made by a processor within controller 270. In some examples, the determination of step 208 may be made by an operator reviewing a representation of the electrical activity (e.g., such as that illustrated in FIG. 4) displayed on interface 280 of FIG. 1.

In the example illustrated in FIG. 4, electrical activity 402 measured between electrode pair 1-2; electrical activity 404 measured between electrode pair 7-8; and electrical activity 408 measured between electrode pair 11-12 may be determined to be the highest electrical activity. As a result, in step 208, electrode pairs 1-2, 7-8, and 11-12 may be determined to be the plurality of electrodes that measured a highest electrical activity. Although described as a pair, any number of two or more electrodes may be used. Upon completion of step 208, method 200 may proceed to step 210.

In some implementations, prior to step 210, an impedance measurement may be made between each adjacent electrode pair (e.g., electrodes 1 and 2, 3 and 4, etc.). These measurements may be stored as "vector impedance" values. Then, after the impedance measurements are recorded, pacing may be delivered to an interior wall of the patient (e.g., bladder wall 110 of FIG. 3) to achieve a muscle/nerve response. Medical device 250 and electrodes 1-20 may have a bipolar configuration. Alternatively, medical device 250 may be a unipolar arrangement. In examples wherein the medical device 250 is a unipolar device, the current would be passed between one of the electrodes and a separate return electrode.

Step 210 may include generating a pacing stimulus from one or more of the plurality electrodes, such as those measuring the highest electrical activity. In the example illustrated in FIG. 4, a pacing stimulus may be generated at electrode pairs 1-2, 7-8, and/or 11-12. The pacing stimulus may be applied at one electrode pair at a time. Generation of a pacing stimulus in step 210 may be initiated by controller 270 or manually by an operator. In one example, controller 270 or a processor within controller 270 may instruct or activate the electrical energy source to generate the pacing stimulus.

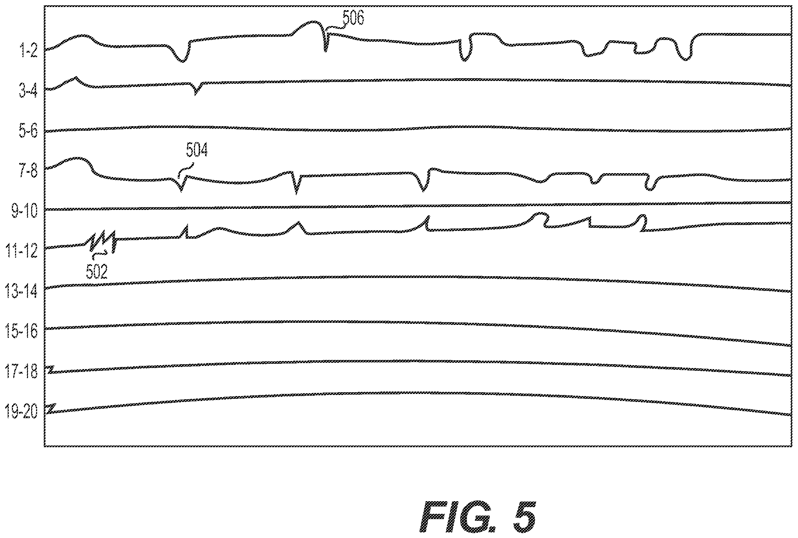

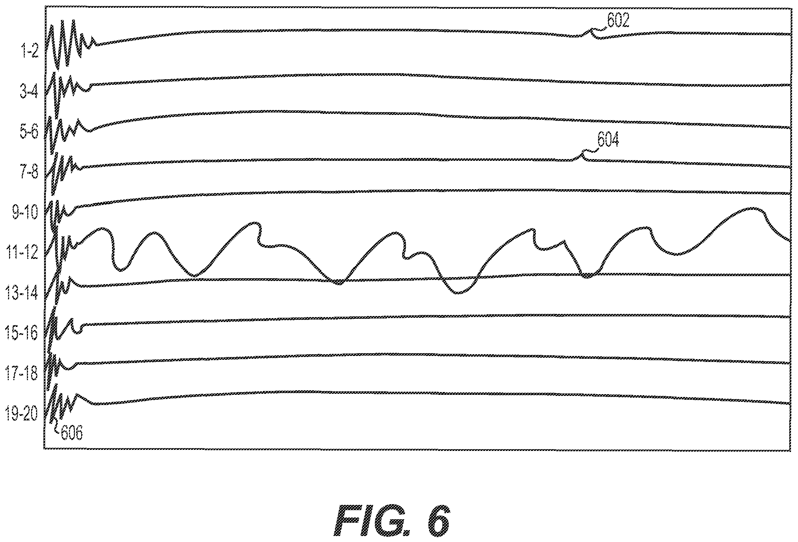

The generated pacing stimulus may be low frequency, high frequency, or both frequencies may be applied sequentially. FIGS. 5 and 6 illustrate graphical representations of exemplary electrical responses to low frequency pacing and high frequency pacing, respectively. Similar to FIG. 4, FIGS. 5 and 6 may be displayed on interface 280 of FIG. 1. The muscle/nerve response may be acquired with a charge-balanced waveform using currents (or voltages) that step up incrementally (e.g. in steps of approximately 0.1 mA (or V) or more). While pacing is occurring between a pair of electrodes, the other electrodes may be in sensing mode, recording EMG responses. These other electrodes may measure electrical activity and communicate resulting electrical signals to controller 270 of FIG.1. For example, in FIGS. 5 and 6, pacing may be occurring at electrodes 11-12 and electrodes 1-10 and 13-20 may measure muscle/nerve responses at various locations on the internal wall. In some examples, the measuring/sensing electrodes may communicate any muscle/nerve response as electrical signals via wires 260 to controller 270 (or the processor within controller 270) of FIG. 1. Controller 270 (or the processor within controller 270) may process these measured electrical signals and/or output the signals for display at interface 280. In some implementations, a map may be created of the voltages required at each electrode pair in order to elicit a muscle response at locations engaged with other electrodes.

FIG. 5 illustrates exemplary registered electrical signal responses at electrodes 1-10 and 13-14 for low frequency pacing 502 applied at electrodes 11-12. Low frequency pacing may be approximately 0.5 Hz to approximately 10 Hz. According to one aspect, low frequency pacing may be approximately 2 Hz. When low frequency pacing is applied, it may be expected that the EMG response from the muscle of the interior wall would occur at multiple locations on the interior wall, as indicated by signal propagations 504 and 506 of FIG. 5. For example, the signal propagation during the pacing procedure may also be presented in a 3 D conduction map.

In some implementations, high frequency pacing (e.g., approximately 100 Hz to approximately 500 Hz and in some instances, approximately 300 Hz) may be applied at an electrode pair instead of low frequency pacing, before low frequency pacing, and/or after low frequency pacing. FIG. 6 illustrates registered exemplary electrical signal responses at electrodes 1-10 and 13-14 for high frequency pacing applied at electrodes 11-12. When high frequency pacing is applied, it may be expected that the EMG response from the nerve stimulation may occur on the order of milliseconds after stimulation (e.g., as illustrated in FIG. 6 by response 606), as the nerve must conduct the signal to the muscle to activate it. High frequency pacing may elicit signals (e.g., signals 602 and 604 of FIG. 6) at other locations (e.g., those engaged with electrodes 1-2 and 7-8).

As shown in FIGS. 5 and 6, the locations where propagation and/or increased signals are detected (e.g., 504, 506, 602, and/or 604) coincide with the locations with the highest electrical activity measured in steps 206 and 208 (e.g., electrode pairs 1-2 and 11-12). This is merely exemplary and locations where propagation and/or signals (e.g., 504, 506, 602, and/or 604) are elicited as a result of pacing may occur at any electrodes, not necessarily electrode pairs 1-2 and 11-12. In some aspects, the pacing may be done before an impedance may be measured. For example, applying stimulation to certain bladder tissues before obtaining the actual impendence measurement may induce stronger signals, such as at highly myogenically active sites.

Pacing (low frequency or high frequency) may be generated at one electrode pair at a time. This electrode pair may be adjacent electrodes or any electrode pair regardless of their distance from each other. In the example shown in FIG. 4, it may be determined in step 208 that three electrode pairs measured the highest electrical activity. Thus, once in step 210, a low frequency and/or high frequency pacing is performed at a first electrode pair (e.g., electrodes 11-12 as illustrated in FIGS. 5 and 6), method 200 may proceed to step 212. In step 212, it may be determined whether a pacing stimulus has been generated from each of the electrodes of the plurality of electrodes that measured the highest electrical activity. If a pacing stimulus has not been generated from each of the electrodes of the plurality of electrodes (Step 212:No), method 200 may proceed to step 210 and generate a pacing stimulus from an electrode of the plurality of electrodes that had not previously generated a pacing stimulus. If a pacing stimulus has been generated from each of the electrodes of the plurality of electrodes (Step 212:Yes), method 200 may proceed to step 214.

In the example illustrated in FIG. 4, electrode pairs 1-2, 7-8, and 11-12 were determined in step 208 to have measured the highest electrical activity. Thus, in the examples illustrated in FIGS. 4-6, since a pacing stimulus is generated from electrode pair 11-12, but not yet electrode pairs 1-2 and 7-8, method 200 may return to step 210 to generate a pacing stimulus from electrode pair 1-2 and/or 7-8. In this example, once a pacing stimulus is generated from each of electrode pairs 1-2, 7-8, and 11-12 (e.g., the electrodes that measure the highest electrical activity in step 208), method 200 may proceed to step 214.

In one example, where low and high frequency pacing may be used, low frequency pacing may first be performed throughout the entire bladder, then high frequency pacing may be performed. Additionally or alternatively, a method may start with high frequency pacing at nerve-rich locations (e.g., the bladder neck and dome) and then create low frequency pacing at other areas in bladder, or vice versa.

In some examples, pacing may be repeated at a given location (e.g., a location adjacent an electrode pair). For example, electrode pair 1-2 may have been paced first and may have resulted in high propagation to other locations. Then, other locations may have been paced. Subsequently, electrode pair 1-2 may be paced a second time to determine if the propagation is observed again. In some examples, electrode pairs may be paced in a random order and not to always paced in the same order. Alternatively, electrode pairs may be paced in the same order every time, starting from one anatomical location to another anatomical location (e.g. from bladder neck to bladder dome, so the method always creates the map in the same way). The various types of pacing described herein may also be repeated after array 116 has been moved from the given location, as noted above.

Step 214 may include, for example, identifying treatment site(s), if any. Identifying treatment site(s) may include creating a map of voltages at each of the plurality of electrodes to elicit a muscle response. This step may be performed manually by an operator, by controller 270, or by a processor within controller 270. According to another aspect of the present disclosure, partial mapping may be performed. In some instances, prolonged pacing may result in changed electrical activity of the bladder, and partial mapping may allow prolonged pacing to be avoided by shortening the mapping steps. For partial mapping, instead of pacing every electrode pair, a location where the most electrical activity is observed (prior to pacing) is first paced. Then, locations that show a subsequent increase in electrical activity are paced. A location that triggers the most activity may be identified as a treatment site and immediately treated. This way, instead of methodically pacing all electrode pairs, a subset of the electrode pairs are paced, whereby information at one electrode pair/site leads to identifying a next electrode pair or location to pace, and then finally to a treatment site.

Referring to FIG. 5, in which a low frequency pacing stimulus is generated at electrode pair 11-12, the treatment site(s) may be those that, when applying a pacing stimulus at one location, elicit the most signal propagation in other locations on the interior wall. Thus, electrodes 11-12 may be identified because applying a pacing stimulus to electrode pair 11-12 caused the most propagation in other locations, for example, propagation 504 and 506 of FIG. 5.

In FIG. 6, in which a high frequency pacing stimulus is generated at electrode pair 11-12, the treatment site(s) may be those that, when applying a pacing stimulus, elicit the most signals from other locations on the interior wall. Again, electrode pair 11-12 may be identified because applying a high frequency pacing stimulus caused signals 602 and 604 in FIG. 6.

In step 216, it may be determined whether there are any treatment sites, e.g., those identified in step 214. If there are treatment sites (Step 216:Yes), method 200 may proceed to step 218. If there are no treatment sites (Step 216:No), method 200 may proceed to step 220.

In step 218, therapy may be applied to the treatment sites. Therapy may include, but is not limited to, application of radio-frequency ("RF") energy, ultrasound energy (e.g., high intensity focused ultrasound), laser energy, cryoablation, microwave ablation, Botox injections, neurolytic agents, optical energy sources, irreversible electroporation, hydrogel injections, and/or other suitable technologies that affect the reactivity of nerve(s). The therapy may also include mucosal resection or similar tissue microdissection and/or cutting. Exemplary optical energy sources may include a holmium (Ho) laser source, a holmium:YAG (Ho:YAG) laser source, a neodymium-doped:YAG (Nd:YAG) laser source, a semiconductor laser diode, a potassium-titanyl phosphate crystal (KTP) laser source, a carbon dioxide (CO₂) laser source, an Argon laser source, an Excimer laser source, and/or a diode laser source. Exemplary neurolytic agents may include ethanol, phenol, glycerol, ammonium salt compounds, chlorocresol and hypertonic and/or hypotonic solutions. In some examples, the same electrodes that deliver the pacing energy may also deliver the treatment. Once step 218 is complete, method 200 may return to step 206. By returning to step 206, it may be determined whether the therapy applied in step 218 was effective, whether more therapy is needed, and/or in what treatment site(s), if any, require additional therapy. This may include determining whether the measured electrical activity falls below a predetermined threshold. If so, additional therapy may be applied.

If there are no treatment sites identified in step 214, method 200 may proceed to step 220. In step 220, it may be determined whether there are locations of the interior wall that the electrodes did not engage. This may include other organs (e.g., kidneys, stomach, intestine) in need of analysis, or locations within the same organ (e.g., bladder 102 of FIG. 3) that had not previously been engaged by electrodes 1-20. If there are locations of the interior wall that the electrodes did not engage (Step 220:Yes), method 200 may proceed to step 222 and the catheter may be moved to a location of the interior wall that the electrodes did not previously engage. Once the catheter is at a location of the interior wall the electrodes did not previously engage, method 200 may return to step 204. If there are no locations of the interior wall that the electrodes did not engage (Step 222:No) or there are no additional locations the operator desires to analyze/treat, method 200 may proceed to step 224 and the catheter may be removed from the patient.

As previously mentioned, method 200 include more or fewer steps than those illustrated in FIG. 2. For example, method 200 may proceed directly from step 206 to step 214 (skipping steps 208, 210, and 212). In such an example, one or more treatment sites may be identified based on the measurement of electrical activity at multiple locations without pacing stimulus. Treatment sites may correspond to the location of electrode pairs which have the highest sum of signal intensity over acquisition time (e.g., hot spots as described above with respect to steps 206 and 208), as this may indicate "overactive" muscle/nerves/tissue. In FIG. 4, the locations which engage electrode pairs 1-2, 7-8, and/or 11-12 may be identified as treatment sites due to high sums of signal intensity received over an acquisition time.

Although described with reference to the highest electrical activity, step 208 may also be utilized to determine one or more electrodes that measure a lowest electrical activity. For example, a pacing stimulus may be generated one electrode pair at a time to identify one or more electrodes measuring a lowest electrical activity. The identified electrodes may coincide with sites having tissue that is fibrosed or denervated and, thus, not capable of generating increased signals. Step 214 may be used to identify these sites. To modify function of a detrusor muscle, for example, the sites identified in 214 may be treated in step 218, e.g., by ablation, hydrogel injection, Botox, etc.

In other implementations, method 200 may not include steps 206 and 208. In such an implementation, the electrodes selected to generate a pacing stimulus (e.g., step 210) may not be determined in step 208 or based on a "heat map." The electrodes that generate a pacing stimulus may be selected at random or in a defined sequence of pacing vectors until treatment sites are determined.

In some implementations, there may be a feedback loop. The feedback loop may adjust, e.g., titrate, the therapy up until the resulting electrical activity meets a predetermined amount or threshold.

In addition, aspects of the aforementioned embodiments may be combined with any other aspects of any other embodiments, without departing from the scope of the disclosure.

Other embodiments of the present disclosure will be apparent to those skilled in the art from consideration of the specification and practice of the embodiments disclosed herein. It is intended that the specification and examples be considered as exemplary only.

The present invention is defined by the following claims.

## Claims

1. A device (250) comprising:
a plurality of electrodes (1-20);
a memory device configured to store an instruction for evaluating electrical activity; and
a processor configured to:
access the instruction from the memory;
measure, with the instruction, a spontaneous electrical activity at the plurality of electrodes (1-20);
identify, with the instruction, a pair of the plurality of electrodes (1-20) with the highest spontaneous electrical activity;
direct, with the instruction, an electrical energy source to generate a pacing stimulus through the previously identified pair of the plurality of electrodes (1-20) with the highest spontaneous electrical activity;
measure, with the instruction, a resulting electrical activity with one or more of the plurality of electrodes (1-20); and
identify, with the instruction, at least one treatment site based on the resulting electrical activity.

2. The device (250) of claim 1, wherein the pacing stimulus is a low frequency pacing stimulus.

3. The device (250) of claim 1, wherein the pacing stimulus is a high frequency pacing stimulus.

4. The device (250) of claim 1, wherein the processor is further configured:
direct, with the instruction, the electrical energy source to generate the pacing stimulus through a first portion of the plurality of electrodes (1-20);
measure, with the instruction, a first resulting electrical activity at the first portion of the plurality of electrodes (1-20); and
identify, with the instruction, a first pair of the plurality of electrodes (1-20) based on the first resulting electrical activity.

5. The device (250) of claim 1, wherein the processor is further configured to initiate, with the instruction, a therapy to the least one treatment site.

6. The device (250) of claim 5, wherein the therapy includes at least one of a radio frequency energy, an ultrasound energy, a laser energy, a cryoablation, a microwave ablation, a Botox injection, a neurolytic agent, an optical energy, an irreversible electroporation, and a hydrogel injection.

7. The device (250) of claim 6, wherein the processor is further configured to modify, with the instructions, the therapy until the resulting electrical activity reaches a predetermined amount.

8. The device (250) of claim 7, wherein the processor is further configured to titrate, with the instructions, the therapy higher until the measured resulting electrical activity reaches a predetermined threshold.

9. The device (250) of claim 1, wherein the processor is further configured to communicate, with the instruction, the resulting electrical activity to a display.

10. The device (250) of claim 9, wherein the processor is further configured to generate, with the instructions, a heat map on the display based on the resulting electrical activity, wherein the heat map is an intensity map created by measuring the sum of signal intensity at each electrode over time,.

11. The device (250) of any preceding claim, wherein the plurality of electrodes (1-20) are engageable with an interior wall of a patient at a plurality of locations.

12. The device (250) of claim 11, wherein the interior wall is a bladder wall.

## Patentansprüche

1. Vorrichtung (250), umfassend:
eine Mehrzahl von Elektroden (1-20);
eine Speichervorrichtung, die dafür ausgelegt ist, einen Befehl für eine Evaluierung einer elektrischen Aktivität zu speichern; und
einen Prozessor, der für Folgendes konfiguriert ist:
Zugreifen auf den Befehl aus dem Speicher;
Messen einer spontanen elektrischen Aktivität an der Mehrzahl von Elektroden (1-20) aufgrund des Befehls;
Identifizieren eines Paars mit der höchsten spontanen elektrischen Aktivität aus der Mehrzahl von Elektroden (1-20) aufgrund des Befehls;
Veranlassen einer Quelle für elektrische Energie, einen Schrittsteuerungsimpuls durch das davor identifizierte Paar mit der höchsten spontanen elektrischen Aktivität aus der Mehrzahl von Elektroden (1-20) zu erzeugen, aufgrund des Befehls;
Messen einer resultierenden elektrischen Aktivität mit einer oder mehreren von der Mehrzahl von Elektroden (1-20) aufgrund des Befehls; und
Identifizieren mindestens einer Behandlungsstelle auf Basis der resultierenden elektrischen Aktivität aufgrund des Befehls.

2. Vorrichtung (250) nach Anspruch 1, wobei der Schrittsteuerungsimpuls ein niederfrequenter Schrittsteuerungsimpuls ist.

3. Vorrichtung (250) nach Anspruch 1, wobei der Schrittsteuerungsimpuls ein hochfrequenter Schrittsteuerungsimpuls ist.

4. Vorrichtung (250) nach Anspruch 1, wobei der Prozessor ferner für Folgendes ausgelegt ist: Veranlassen der Quelle für elektrische Energie, den Schrittsteuerungsimpuls durch einen ersten Teil von der Mehrzahl von Elektroden (1-20) zu erzeugen, aufgrund des Befehls;
Messen einer ersten resultierenden elektrischen Aktivität an dem ersten Teil von der Mehrzahl von Elektroden (1-20) aufgrund des Befehls; und
Identifizieren eines ersten Paars von der Mehrzahl von Elektroden (1-20) auf Basis der ersten resultierenden elektrischen Aktivität aufgrund des Befehls.

5. Vorrichtung (250) nach Anspruch 1, wobei der Prozessor ferner dafür ausgelegt ist, aufgrund des Befehls eine Therapie für die mindestens eine Behandlungsstelle zu initiieren.

6. Vorrichtung (250) nach Anspruch 5, wobei die Therapie mindestens eine(s) von einer Hochfrequenzenergie, einer Ultraschallenergie, einer Laserenergie, einer Kryoablation, einer Mikrowellenablation, einer Botoxinjektion, einem neurolytischen Wirkstoff, einer optischen Energie, einer irreversiblen Elektroporation und einer Hydrogelinjektion einschließt.

7. Vorrichtung (250) nach Anspruch 6, wobei der Prozessor ferner dafür ausgelegt ist, aufgrund der Befehle die Therapie zu modifizieren, bis die resultierende elektrische Aktivität eine vorgegebene Stärke erreicht.

8. Vorrichtung (250) nach Anspruch 7, wobei der Prozessor ferner dafür ausgelegt ist, aufgrund der Befehle die Therapie höher zu titrieren, bis die gemessene resultierende elektrische Aktivität einen vorgegebenen Schwellenwert erreicht.

9. Vorrichtung (250) nach Anspruch 1, wobei der Prozessor ferner dafür ausgelegt ist, aufgrund des Befehls die resultierende elektrische Aktivität an eine Anzeige zu übermitteln.

10. Vorrichtung (250) nach Anspruch 9, wobei der Prozessor ferner dafür ausgelegt ist, mittels der Befehle auf Basis der resultierenden elektrischen Aktivität ein Heatmap auf der Anzeige anzuzeigen, wobei das Heatmap ein Karte von Intensitäten ist, die durch Messen der Summe der Signalstärke an jeder Elektrode im Zeitverlauf erzeugt wird.

11. Vorrichtung (250) nach einem der vorangehenden Ansprüche, wobei die Mehrzahl von Elektroden (1-20) an einer Innenwand eines Patienten an einer Mehrzahl von Stellen anbringbar ist.

12. Vorrichtung (250) nach Anspruch 11, wobei die Innenwand eine Blasenwand ist.

## Revendications

1. Dispositif (250) comprenant :
une pluralité d'électrodes (1 - 20) ;
un dispositif de mémoire qui est configuré de manière à ce qu'il stocke une instruction pour évaluer une activité électrique ; et
un processeur qui est configuré de manière à ce qu'il réalise les actions qui suivent :
l'accès à l'instruction à l'intérieur de la mémoire ;
la mesure, au moyen de l'instruction, d'une activité électrique spontanée au niveau des électrodes de la pluralité d'électrodes (1 - 20) ;
l'identification, au moyen de l'instruction, d'une paire d'électrodes de la pluralité d'électrodes (1 - 20) présentant l'activité électrique spontanée la plus élevée ;
la commande, au moyen de l'instruction, d'une source d'énergie électrique de manière à ce qu'elle génère un stimulus de stimulation au travers de la paire d'électrodes identifiée au préalable de la pluralité d'électrodes (1 - 20) présentant l'activité électrique spontanée la plus élevée ;
la mesure, au moyen de l'instruction, d'une activité électrique résultante avec une ou plusieurs électrode(s) de la pluralité d'électrodes (1- 20) ; et
l'identification, au moyen de l'instruction, d'au moins un site de traitement sur la base de l'activité électrique résultante.

2. Dispositif (250) selon la revendication 1, dans lequel le stimulus de stimulation est un stimulus de stimulation basse fréquence.

3. Dispositif (250) selon la revendication 1, dans lequel le stimulus de stimulation est un stimulus de stimulation haute fréquence.

4. Dispositif (250) selon la revendication 1, dans lequel le processeur est en outre configuré de manière à ce qu'il réalise les actions qui suivent : la commande, au moyen de l'instruction, de la source d'énergie électrique de manière à ce qu'elle génère le stimulus de stimulation par l'intermédiaire et au travers d'une première partie de la pluralité d'électrodes (1 - 20) ;
la mesure, au moyen de l'instruction, d'une première activité électrique résultante au niveau de la première partie de la pluralité d'électrodes (1 - 20) ; et
l'identification, au moyen de l'instruction, d'une première paire d'électrodes de la pluralité d'électrodes (1 - 20) sur la base de la première activité électrique résultante.

5. Dispositif (250) selon la revendication 1, dans lequel le processeur est en outre configuré de manière à ce qu'il initie, au moyen de l'instruction, une thérapie sur l'au moins un site de traitement.

6. Dispositif (250) selon la revendication 5, dans lequel la thérapie inclut au moins un moyen thérapeutique pris parmi une énergie radiofréquence, une énergie ultrasonore, une énergie laser, une cryoablation, une ablation hyperfréquence, une injection de botox, un agent neurolytique, une énergie optique, une électroporation irréversible et une injection d'hydrogel.

7. Dispositif (250) selon la revendication 6, dans lequel le processeur est en outre configuré de manière à ce qu'il modifie, au moyen de l'instruction, la thérapie jusqu'à ce que l'activité électrique résultante atteigne un degré prédéterminé.

8. Dispositif (250) selon la revendication 7, dans lequel le processeur est en outre configuré de manière à ce qu'il ajuste, au moyen de l'instruction, la thérapie selon un degré plus élevé jusqu'à ce que l'activité électrique résultante mesurée atteigne un seuil prédéterminé.

9. Dispositif (250) selon la revendication 1, dans lequel le processeur est en outre configuré de manière à ce qu'il communique, au moyen de l'instruction, l'activité électrique résultante à un affichage.

10. Dispositif (250) selon la revendication 9, dans lequel le processeur est en outre configuré de manière à ce qu'il génère, au moyen de l'instruction, une carte thermique sur l'affichage sur la base de l'activité électrique résultante, dans lequel la carte thermique est une carte d'intensité qui est créée en mesurant la somme des intensités de signal au niveau de chacune des électrodes au fil du temps.

11. Dispositif (250) selon l'une quelconque des revendications précédentes, dans lequel les électrodes de la pluralité d'électrodes (1 - 20) peuvent être engagées avec une paroi intérieure d'un patient au niveau d'une pluralité de localisations.

12. Dispositif (250) selon la revendication 11, dans lequel la paroi intérieure est une paroi de la vessie.
